# EUROPÄISCHE PATENTSCHRIFT

(11) **EP 1 625 872 B1**
(45) Veröffentlichungstag und Bekanntmachung des Hinweises auf die Patenterteilung: **12.03.2008**
(21) Anmeldenummer: 05016164.5
(22) Anmeldetag: 26.07.2005
(51) Int. Cl.: A61M 39/00

(54) **Luer-Lock-Verbinder für medizinische Geräte**
Luer-lock connector for medical devices
Connecteur du type luer-lock pour dispositifs médicaux

(30) Priorität: 12.08.2004 DE 202004012714 U
(43) Veröffentlichungstag der Anmeldung: 15.02.2006
(73) Patentinhaber: Smiths Medical Deutschland GmbH, 85614 Kirchseeon (DE)
(72) Erfinder: Jörg Weber, 83533 Edling (DE); Bernd Beck, 72414 Rangendingen (DE)
(74) Vertreter: von Bülow, Tam

(56) Entgegenhaltungen:
- WO-A-20/04027305
- US-A- 4 360 024
- US-A- 4 452 473
- US-A1- 2002 115 984

## Beschreibung

Die Erfindung bezieht sich auf einen Luer-Lock-Verbinder für medizinische Geräte gemäß dem Oberbegriff des Patentanspruches 1.

Ein solcher Luer-Lock-Verbinder ist aus der DE 103 03 381 A1 bekannt. Dieser Luer-Lock-Verbinder hat, wie auch sonst in der Medizintechnik allgemein üblich, einen rohrförmigen Körper und eine diesen umgebenden und an ihm gehaltene Überwurfmutter mit einem Innengewinde, die in ein Außengewinde eines Luer-Lock-Gegen-Elementes eingreift. Üblicherweise ist die Überwurfmutter an dem sog. männlichen Luer-Lock-Teil angebracht. Bei der Montage wird sie vom freien Ende des rohrförmigen Körpers her aufgeschoben und wird dabei zwangsläufig um ein gewisses Maß geweitet. Selbst wenn man noch vollständig im elastischen Bereich bleibt, so kann die Überwurfmutter nach der Montage auch wieder durch entsprechende Dehnung heruntergezogen werden, so daß eine unlösliche Fixierung der Überwurfmutter nach dem Aufschieben kaum möglich ist.

Um ein Herunterrutschen der Überwurfmutter bei Zugbeanspruchung, wie sie bei einer Verschraubung auftritt, zu vermeiden, wird an der Überwurfmutter und dem rohrförmigen Körper jeweils eine Kante angebracht, wobei die beiden Kanten als Anschlag gegen ein Herunterziehen dienen. Solche Kanten können zwar in der Praxis die Überwurfmutter relativ gut halten. Falls aber beim Aufschieben der Überwurfmutter das Material plastisch gedehnt wurde, wird diese Kante keine sichere Befestigung der Überwurfmutter gewährleisten. Ein weiterer Nachteil einer solchen Kante ist, daß die Überwurfmutter beliebig weitergedreht werden kann, da sie auf keinen Konus gezogen wird, der zu einer Verriegelung bzw. Fixierung der Überwurfmutter führen würde. Bei einem Weiterdrehen der Überwurfmutter wird der weibliche Luer-Connector immer weiter auf den männlichen Luer-Kegel gezogen. Dadurch entstehen im weiblichen Luer-Connector erhebliche Spannungen, was schnell zu Materialversagen, wie Spannungsrissen oder Undichtigkeiten, führen kann. Ein weiterer Nachteil einer Kante ist, daß bei nicht ausreichendem Festziehen der Überwurfmutter oder bei zu glatten Oberflächen der Gewinden die Gefahr besteht, daß sich die Überwurfmutter selbsttätig zurückdreht und sich die gesamte Verbindung lockert. Dies wird noch durch Gleitmittel oder Flüssigkeiten begünstigt.

Bei der DE 103 03 381 A1 ist zwischen der Überwurfmutter und dem männlichen Luer-Kegel ein Konus vorhanden, der beim Aufschieben der Überwurfmutter und vor allem deren radial nach innen ragende Kante eine Aufweitung bewirkt und somit das Risiko vergrößert, daß die Überwurfmutter vom rohrförmigen Körper abgezogen werden kann.

Ein Luer-Lock-Verbinder, bei dem zwei relativ zueinander drehbare Teile durch Anschlagkanten in Axialrichtung relativ zueinander fixiert werden, ist auch aus der US 6,364,869 B1 bekannt. Auch hier ist aber eine Schräge vorhanden, die beim Zusammenstecken der Teile eine Aufweitung des äußeren Teiles bewirken kann.

Aufgabe der Erfindung ist es daher, einen Luer-Lock-Verbinder zu schaffen, der eine sichere Halterung der Überwurfmutter an dem rohrförmigen Körper gewährleistet, obwohl die Montage ebenfalls durch ein Zusammenstecken von Überwurfmutter und rohrförmigem Körper erfolgt.

Diese Aufgabe wird durch die im Patentanspruch 1 angegebenen Merkmale gelöst. Vorteilhafte Ausgestaltungen und Weiterbildungen der Erfindung sind den Unteransprüchen zu entnehmen.

Das Grundprinzip der Erfindung besteht darin, daß mindestens ein Federelement vorgesehen ist, das sich beim Zusammenstecken der beiden Teile elastisch verformt und nach dem Zusammenstecken aufweitet und als Anschlag gegen ein Herunterrutschen der Überwurfmutter vom rohrförmigen Körper dient.

Vorzugsweise werden zwei oder mehrere federelastische Arme verwendet, die am rohrförmigen Körper angebracht sind und radial nach außen vorspringend angeordnet sind. Ihre Stirnseite kommt mit einer Kante an der Überwurfmutter in Anschlag.

Dabei kann zwischen Überwurfmutter und rohrförmigem Körper ein Konussitz vorgesehen sein. Selbst wenn dieser bei dem Zusammenstecken der beiden Teile aufgeweitet wird, so weiten sich die federelastischen Arme doch wesentlich weiter auf und kommen stets mit Sicherheit in Anschlag mit der Kante.

Im folgenden wird die Erfindung anhand eines Ausführungsbeispieles im Zusammenhang mit der Zeichnung ausführlicher erläutert. Es zeigt:

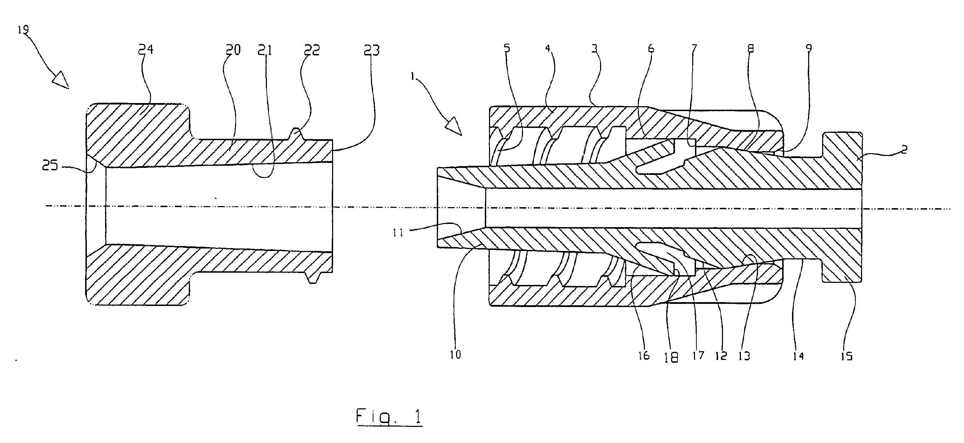

- Fig. 1: einen Längsschnitt eines Luer-Lock-Verbinders nach der Erfindung.

Der Luer-Lock-Verbinder hat einen ersten Verbinderteil, der üblicherweise als männlicher Luer-Lock-Verbinder bezeichnet wird und das Bezugszeichen 1 trägt. Dieser Verbinder hat einen rohrförmigen Körper 2 und eine diesen teilweise umgebende drehbare Überwurfmutter 3, die einen aufgeweiteten Teil 4 mit größerem Durchmesser hat, der mit einem Innengewinde 5 versehen ist. Hieran schließt sich ein verengter Teil 6 mit geringerem Durchmesser an, der an einer radial nach innen vorspringenden Kante 7 endet. Von dort schließt sich ggf. noch über einen zylindrischen Teil ein Dichtungskonus 8 an, der fortschreitend zu dem dem Gewinde 5 abgewandten Ende seinen Durchmesser verringert, bis er über eine Fase 9 zum hinteren Ende der Überwurfmutter 3 mündet.

Der rohrförmige Körper 2 hat an seinem vorderen Ende einen Luer-Konus 10, an dessen vorderem Ende ein Innenkonus 11 vorhanden ist. Am hinteren Ende des Luer-Konus 10 sind hier mindestens zwei federelastische Arme 16 angebracht, die radial nach außen vorspringen. Ihre hintere Stirnkante 18 bildet dabei einen Anschlag mit der Kante 7 der Überwurfmutter. Zwischen dem Material des rohrförmigen Körpers 2 und den federelastischen Armen 16 ist somit ein Ringraum 17 gebildet, in welchen die federelastischen Arme 16 hineinfedern können. An diesen Ringraum schließt sich über eine Schräge 12 ein Dichtungskonus 13 an, der an den Dichtungskonus 8 der Überwurfmutter angepaßt ist. An diesen Konus 13 schließen sich ein zylindrischer Abschnitt 14 und ein Endstück 15 an.

Bei der Montage wird der rohrförmige Körper 2 mit dem freien Ende des Luer-Konus 10 in das hintere Ende der Überwurfmutter an der Fase 9 eingeschoben. Die federelastischen Arme 16 können beim Vorbeigleiten an dem Konus 13 nach innen in den Ringraum 17 hineinfedern und, wenn sie an der Kante 7 vorbeigeglitten sind, nach außen gegen die Innenwandung des verengten Teiles 6 ausfedern. Beim weiteren Einführen wird der Konus 8 der Überwurfmutter 3 durch die Schräge 12 und den Konus 13 aufgeweitet, bis die beiden Teile ineinander geschoben sind. Selbst wenn hierbei eine plastische, also bleibende Verformung, d.h. Aufweitung der Überwurfmutter stattfindet, so ist die Kante 7 immer noch wirksam als Anschlag für die Stirnkante 18 der federelastischen Arme 16, so daß selbst nach einer Aufweitung eine sichere und unlösbare Verbindung zwischen der Überwurfmutter und dem rohrförmigen Körper gegeben ist.

Der Vollständigkeit halber ist in Fig. 1 auch das Luer-Lock-Gegenstück 19 dargestellt, das als weibliches Luer-Lock-Teil bezeichnet wird. Es hat ebenfalls einen rohrförmigen Körper 20 mit einem Innenkonus 21, der an den Luer-Konus 10 angepaßt ist. An seinem Außenumfang ist ein Außengewinde 22 vorgesehen, das mit dem Innengewinde 5 der Überwurfmutter zusammenwirkt. Eine Vorderkante 23 des Gegenstückes bildet dessen vorderes Ende. Da die beiden Konuse 10 und 21 ineinandergreifen, kann beim Verschrauben die Überwurfmutter 3 nach vorne in Richtung zum freien Ende des Luer-Konus 10 gezogen werden, womit einerseits die Dichtung zwischen dem Konus 8 und dem Konus 13 wirksam wird, andererseits aber auch eine Aufweitung zwischen diesen beiden Konusen erfolgen kann. Hier setzt dann aber der Anschlag zwischen den federelastischen Armen 16 und der Kante 7 ein, der ein weiteres Vorschieben und damit ein Abziehen der Überwurfmutter verhindert.

Das weibliche Luer-Lock-Teil 19 hat noch ein verbreitertes Greiferteil 24 sowie an seinem hinteren Ende eine Fase 25 für den Anschluß eines medizinischen Gerätes, eines medizinischen Schlauches oder sonstigen Gegenstandes.

## Patentansprüche

1. Luer-Lock-Verbinder für medizinische Geräte mit einem rohrförmigen Körper (2), welcher ein vorderes distales Ende mit Luer-Konus (10) aufweist, und einer diesen Körper umgebenden und an ihm gehaltenen Überwurfmutter (3), wobei
die Überwurfmutter eine radial nach innen vorspringende Kante (7) aufweist, und **dadurch gekennzeichnet, daß** der rohrförmige Körper (2) mindestens einen radial nach außen vorspringenden federelastischen Arm (16) aufweist, dessen Stirnseite (18) mit der Kante (7) in Anschlag bringbar ist.

2. Luer-Lock-Verbinder nach Anspruch 1, **dadurch gekennzeichnet,**
**daß** der rohrförmige Körper (2) zwei oder mehr federelastische Arme (16) aufweist.

3. Luer-Lock-Verbinder nach Anspruch 1 oder 2, **dadurch gekennzeichnet,**
**daß** die Überwurfmutter (3) und der rohrförmige Körper (2) je einen Dichtungskonus (8 bzs. 13) aufweisen, die aneinander angepaßt sind.

## Claims

1. Luer-Lock connector for medical devices comprising:
a tubular body (2) having a forward distal end with a Luer cone (10) and a swivel nut (3) enclosing this body and secured thereon, wherein the swivel nut has a radially inward-protruding edge (7), **characterized in that** the tubular body (2) comprises at least one radially outward-protruding spring elastic arm (16) whose front face (18) can be brought against a stop with the edge (7).

2. Luer-Lock connector according to claim 1, **characterized in that** the tubular body (2) comprises two or more spring elastic arms (16).

3. Luer-Lock connector according to claim 1 or 2, **characterized in that** the swivel nut (3) and the tubular body (2) each have a sealing cone (8, 13) adapted to each other.

## Revendications

1. Raccord luer-lock pour instruments médicaux, comportant un corps tubulaire (2), lequel comporte une extrémité distale avant avec un cône luer (10), et un écrou d'accouplement (3) entourant ledit corps et maintenu contre celui-ci, ledit écrou d'accouplement comportant un bord (7) en saillie radiale vers l'intérieur, et **caractérisé en ce que** le corps tubulaire (2) comporte au moins un bras (16) élastique, en saillie radiale vers l'extérieur, dont la face frontale (18) peut être amenée en butée contre le bord (7).

2. Raccord luer-lock selon la revendication 1, **caractérisé en ce que** le corps tubulaire (2) comporte deux ou plusieurs bras (16) élastiques.

3. Raccord luer-lock selon la revendication 1 ou 2, **caractérisé en ce que** l'écrou d'accouplement (3) et le corps tubulaire (2) comportent chacun un cône d'étanchéité (respectivement 8 et 13), qui sont adaptés l'un à l'autre.
